(19) **Europäisches Patentamt**
**European Patent Office**
**Office européen des brevets**

(11) **EP 2 531 263 B1**

(12) **EUROPEAN PATENT SPECIFICATION**

(45) Date of publication and mention
of the grant of the patent:
**22.06.2016 Bulletin 2016/25**

(21) Application number: **11700549.6**

(22) Date of filing: **18.01.2011**

(51) Int Cl.:
*A61K 8/35* (2006.01)    *A61K 8/06* (2006.01)
*A61Q 17/04* (2006.01)    *A61K 8/31* (2006.01)
*A61K 8/34* (2006.01)    *A61K 8/36* (2006.01)
*A61K 8/37* (2006.01)    *A61K 8/86* (2006.01)
*A61K 8/891* (2006.01)

(86) International application number:
**PCT/EP2011/050620**

(87) International publication number:
**WO 2011/095392 (11.08.2011 Gazette 2011/32)**

(54) **A PHOTOSTABLE SUNSCREEN COMPOSITION**

LICHTSTABILE SONNENABSCHIRMUNGSZUSAMMENSETZUNG

COMPOSITION D'ÉCRAN SOLAIRE PHOTOSTABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.02.2010 IN MU02812010**

(43) Date of publication of application:
**12.12.2012 Bulletin 2012/50**

(73) Proprietors:
• **Unilever N.V.
3013 AL Rotterdam (NL)**
Designated Contracting States:
**AL AT BE BG CH CZ DE DK EE ES FI FR GR HR
HU IS IT LI LT LU LV MC MK NL NO PL PT RO RS
SE SI SK SM TR**
• **Unilever PLC
London, Greater London EC4Y 0DY (GB)**
Designated Contracting States:
**CY GB IE MT**

(72) Inventors:
• **DUGGAL, Charu
Whitefield Bangalore 560066 (IN)**
• **RAUT, Janhavi, Sanjay
Whitefield Bangalore 560066 (IN)**

(74) Representative: **Corsten, Michael Allan
Unilever N.V.
Unilever Patent Group
Olivier van Noortlaan 120
3133 AT Vlaardingen (NL)**

(56) References cited:
**EP-A2- 1 092 414    WO-A1-01/37798
US-A- 5 576 354    US-A- 5 985 251**

## Description

### Technical Field

The present invention relates to photostable sunscreen compositions comprising sunscreens, more particularly to a composition in microemulsion format comprising dibenzoylmethane sunscreens.

### Background of the invention

Solar radiation includes about 5% ultraviolet (UV) radiation, wavelength of which is between 200nm and 400 nm. It is further classified into three regions: from 320 to 400 nm (UV-A), 290 to 320 nm (UV-B) and from 200 to 290 nm (UV-C). A large part of UV-C radiation is absorbed by the ozone layer. Scientific studies have indicated that exposure to UV-A and UV-B radiation for short period causes reddening of the skin and localized irritation, whereas continued and prolonged exposure can lead to sunburn, melanoma and formation of wrinkles. It is also reported that UV radiation causes significant damage to hair. Therefore, it is desirable to protect the skin and other keratinous substrates of the human body from the harmful effects of both, UV-A and UV-B radiation.

Various cosmetic preparations have been reported for preventing and/or protecting the skin from harmful effects of ultraviolet radiation. Numerous organic sunscreen agents capable of absorbing UV-A rays are reported in the field of cosmetics amongst which a particularly useful sunscreen is of the dibenzoylmethane class. p-Methoxycinnamic acid and its derivatives are used extensively as UV-B sunscreens. Many cosmetic manufacturers prefer to include both UV-A and UV-B sunscreens in photoprotective compositions so as to provide protection over the entire range of UV radiation.

It is known that dibenzoylmethane and its derivatives are relatively sensitive to ultraviolet radiation and they decompose rapidly under the effect of sunlight. This decomposition is accelerated in the presence of UV-B sunscreens, especially p-methoxycinnamic acid and its derivatives. Owing to photochemical instability of dibenzoylmethane and its derivatives in the presence of UV-B sunscreens, especially p-methoxycinnamic acid and its derivatives, one cannot guarantee constant protection during prolonged exposure to the sun. This therefore warrants repeated applications at regular and frequent intervals by the user in order to maintain effective protection against UV rays.

Stabilization of dibenzoylmethane and its derivatives therefore is important so that the user gets complete advantage of its efficacy and he does not have to resort to frequent applications.

Various methods have been reported for stabilization of dibenzoylmethane and its derivatives in cosmetic formulations.

US 5 985 251 (Roche Vitamins, 1999) describes light screening cosmetics wherein compositions comprising dibenzoylmethane derivatives and p-methoxycinnamic acid derivatives are stabilized by incorporating 0.5 to 12% by weight 3,3-diphenylacrylate derivatives or benzylidene camphor derivatives.

US5576354 (L'Oreal, 1996) discloses a photostable cosmetic screening composition and process for protecting of the human epidermis against UV rays of wavelengths 280 to 380 nm, the composition having at least one fatty phase, 1 to 5% by weight of a dibenzoyl methane derivative and at least 1% by weight of an alkyl β-β-diphenylacrylate or α-cyano-β-β-diphenylacrylate of a given formula, the mole ratio of the compound of given formula to the dibenzoylmethane being not less than 0.8.

3,3-diphenylacrylate derivatives or benzylidene camphor derivatives are themselves sunscreens which are expensive and so add to the cost of the compositions. Researchers, including the present inventors, have therefore been looking for simpler solutions to the problem which may involve using combination of more commonly available ingredients to solve this problem.

WO 01/37798 is concerned with the problem of the photostability of sunscreen compositions and discloses compositions containing dibenzoylmethane and p-methoxycinnamate as UV-filters and a mixture of emulsifiers and oil components.

Therefore there exists the need for cosmetic compositions comprising dibenzoylmethane or its derivatives, which are stable, especially in the presence of p-methoxycinnamic acid or its derivatives, wherein specialty polymers and/or additional sunscreen stabilizers are not essentially required. It is highly desirable to have cosmetic compositions, which are stabilized with ingredients that are readily available, thereby reducing the complexities of formulation and substantially reduce costs.

The present applicants have in the past worked on one approach which is published as WO 2008/022946 which discloses cosmetic compositions comprising dibenzoylmethane or its derivative and p-methoxycinnamic acid or its derivatives which can be stabilized by incorporating a combination of fatty alcohol ethoxylates and polyalkyleneglycol. The present inventors have surprisingly found that cosmetic compositions comprising dibenzoylmethane or its derivative can be stabilized in a specific microemulsion formulation having specified ingredients in selective amounts.

It is therefore an object of the present invention to obviate at least some drawbacks of the prior art and provide photostable cosmetic sunscreen compositions.

Another object of the present invention is to provide photostable compositions comprising dibenzoylmethane sunscreens, wherein the stabilization is brought about by using readily available and inexpensive ingredients.

## Summary of the invention

According to one aspect, the present invention relates to a photostable sunscreen composition in microemulsion format with disposed phase of droplets in the size range of 4 to 100 nm. The photostable sunscreen composition comprising:

   a. 0.1 % to 10 % by weight dibenzoylmethane or its derivative;
   b. 10 to 40% by weight an emulsifier;
   c. 5 to 40% by weight a co-emulsifier selected from the group consisting of C2 to C8 straight or branched chain alcohol, polyhydric alcohol and C5 to C8 low molecular weight fatty acid;
   d. 10 to 50% by weight of a hydrophobic liquid having a log P value at 25 degrees centigrade greater than 3 and a water solubility at 25 degrees centigrade less than 0.1% by weight; and
   e. 10 to 50% by weight water,

wherein said emulsifier is a nonionic surfactant;
wherein the dibenzoylmethane derivative is selected from 4-tert-butyl-4'-methoxydibenzoylmethane, 2-methyldibenzoylmethane, 4-methyl-dibenzoyl-methane, 4-isopropyldibenzoylmethane, 4-tert-butyldibenzoylmethane, 2, 4-dimethyldibenzoylmethane, 2, 5-dimethyldibenzoylmethane, 4, 4'-diisopropyl-dibenzoylmethane, 2-methyl-5-isopropyl-4'-methoxydibenzoylmethane, 2-methyl-5-tert-butyl-4'-methoxydibenzoylmethane,2, 4-dimethyl-4'-methoxydibenzoylmethane or 2, 6-dimethyl-4-tert-butyl-4'-methoxy-dibenzoylmethane.

## Detailed Description of the Invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

By "Photoprotective Sunscreen Composition" as used herein, is meant to include a composition for topical application to sun-exposed areas of the skin and/or hair of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, soap bar or toner, or applied with an implement or via a face mask, pad or patch. Non-limiting examples of photoprotective sunscreen compositions include leave-on skin lotions and creams, shampoos, conditioners, shower gels, toilet bars, antiperspirants, deodorants, lipsticks, foundations, mascara, sunless tanners and sunscreen lotions. "Skin" as used herein is meant to include skin on the face and body (e.g., neck, chest, back, arms, underarms, hands, legs, buttocks and scalp) especially to the sun exposed parts thereof. The composition of the invention is also of relevance to applications on any other keratinous substrate of the human body other than skin e.g. hair where products may be formulated with specific aim of providing photoprotection.

By 'photostable sunscreen composition' is meant a composition that is more stable than similar sunscreen compositions known heretofore and/or provides such photostability at lower cost or with ingredients more compatible in the composition. It is preferred that the photostability is such that dibenzoylmethane is preferably present in at least 65%, further more preferably at least 70% after exposure to sunlight for 30 minutes.

The present invention relates to a sunscreen composition in microemulsion format comprising The components specified in present claim 1.

The composition of the present invention is in the form of a microemulsion. Microemulsions are defined as single phase, thermodynamically stable isotropic solutions of water, oil and amphiphiles. They are characterized by an ultralow interfacial tension and a flexible oil-water interface. These can be met by appropriate choice of the surfactants and co-surfactants for a given oil-water system. Microemulsions form spontaneously upon simple mixing of the ingredients and do not require high shear or energy input. Because of the highly flexible interface, microemulsions can evolve between various structures ranging from droplet-like swollen micelles to bicontinuous structures. Bi-continuous microemulsions render the usual "oil in water" and "water in oil" distinction irrelevant. The droplet size in a microemulsion is usually in the size range of 10-100 nm. Thus the microemulsions appear transparent because the droplet size is smaller than the wavelength of visible light.

The composition of the invention is in the microemulsion form and it is preferably transparent. The transparent liquid microemulsion format of the composition is especially preferred since the transparent form makes for an appealing visual impact on consumers. By the term transparent or translucent composition is meant that the composition has a turbidity value of 0 to 200 Nephelometric Turbidity Units (NTU) preferably less than 100 NTU, more preferably less than 10 NTU. The turbidity in units of NTU is as measured using a Merck Turbiquant 1500T turbidity meter. Turbidity is the cloudiness or haziness of a fluid caused by the presence of individual suspended particles (solid) or droplets (liquid) which differ in their optical properties from the suspending fluid. The extent of scattering of a light beam by the suspended particles/droplets is considered a meaningful measure of turbidity. Turbidity measured this way uses an instrument called a nephelometer with the detector setup to the side of the light beam. The amount of light reaching the detector is higher if the extent of scattering from the suspended particles is higher. The units of turbidity from a calibrated nephelometer are termed Nephelometric Turbidity Units (NTU). The extent of light scattered by the suspended particles/droplets is a function of their concentration, size, shape and relative optical properties. More importantly, the microemulsion form of the sunscreen composition of the invention is seen to be more stable than the emulsion form. In a preferred aspect, the dispersed phase droplets in the microemulsion are in the size range of 4 to 100 nm, more preferably in the size range of 4 to 50 nm.

The composition of the invention comprises 0.1 to 10% by weight dibenzoylmethane or its derivative **selected from** 4-tert-butyl-4'-methoxydibenzoylmethane, 2-methyldibenzoylmethane, 4-methyl-dibenzoylmethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoylmethane, 2,4-dimethyl-4'-methoxydibenzoylmethane or 2,6-dimethyl-4-tert-butyl-4'-methoxy-dibenzoylmethane. The most preferred dibenzoylmethane derivative is 4-tert.-butyl-4'-methoxydibenzoylmethane. Dibenzoylmethane or its derivative is preferably present in 0.1 to 5%, more preferably 0.2 to 5%, further more preferably 0.4 to 3% by weight of the composition.

The composition of the invention comprises a 10 to 40%, more preferably 10 to 30% by weight of an emulsifier which is a surfactant i.e a surface active agent which reduces the surface tension when dissolved in water down from a value of about 72 dynes/cm$^2$ to less than 10 dynes/cm$^2$ more preferably less than 5 dynes/cm$^2$. The emulsifier is a surfactant of the non-ionic type. Preferred non-ionic surfactant for use in the present invention is selected from polyethylene glycol monoethyl ether (Brij), polysorbate (Tween), alcohol ethoxylates, or polyoxyethylene esters of fatty acids. It is preferred that the surfactant which acts as the emulsifier in the present invention has an HLB value greater than 8.

The composition of the invention comprises 5 to 40%, preferably 5 to 30% by weight a co-emulsifier selected from C2 to C8 straight or branched chain alcohol, polyhydric alcohol or C5 to C8 low molecular weight fatty acids. More preferred co-emulsifier is selected from short chain alcohols, diols, polyols or fatty acids. Most preferred co-emulsiers are ethanol, propanol, butanol, polyethylene glycol, propylene glycol or caprylic acid.

The composition of the invention comprises 10 to 50%, preferably 10 to 40% by weight of a hydrophobic liquid having a log P value at 25 degrees centigrade greater than 3 and a water solubility at 25 degrees centigrade less than 0.1% by weight. Log P is a property defined in the article Hiroshi Chuman, Atsushi Mori and Hideji Tanaka, "Prediction of the 1-Octanol/H2O Partition Coefficient, Log P, by Ab Initio MO Calculations: Hydrogen-Bonding Effect of Organic Solutes on Log P", Analytical Sciences, September 2002, Vol. 18, 1015-1020. Log P is basically the 1-octanol/water partition coefficient and is experimentally determined by the shake-flask method.

The hydrophobic liquids having a log P value at 25 degrees centigrade greater than 3.0 and water solubility at 25 degrees centigrade less than 0.1% by weight include hydrocarbons for e.g. octanes, nonane, decane, dodecane, hexadecane, paraffin oil; alcohols for e.g. dodecanol, oleyl alcohol; acids for e.g oleic acid; aldehydes for e.g. dodecanal, hexadecanal; ketones for e.g. methyl nonyl ketone; esters for e.g. methyl stearate, eicosanoic acid methyl ester, palm fatty acid ester; ethers for e.g. di iso pentyl ether, dihexyl ether, diheptyl ether, dioctyl ether, didecyl ether. The more preferred hydrophobic liquid for use in the composition of the invention are hydrocarbons, medium or long chain fatty alcohols, fatty alcohol esters, triglycerides, fatty acids or silicone oils. The most preferred hydrophobic liquid as per the present invention are LLPO (light liquid paraffin oil), IPM (iso propyl myristate), IPP (iso propyl palmitate) or silicone oil.

The water solubility at 25 degrees centigrade of the hydrophobic liquid is preferably less than 0.01% and more preferably less than 0.001% by weight.

The composition comprises 10 to 50%, preferably 10 to 40% by weight water. The use of emulsifiers, co-emulsifiers, hydrophilic liquid and water is well known in preparing microemulsions. However, the surprising element in the present invention is that specific microemulsions and specific emulsions close to the microemulsion boundaries result in enhanced stability of dibenzoylmethane compounds and their derivatives when present in them.

The composition additionally may comprise other UV-A or UV-B sunscreens or sunblocks to provide more compete sun protection. Of these, at least one UV-B sunscreen and at least one inorganic sunblock is usually incorporated in a photoprotective sunscreen composition. A preferred UV-B sunscreen is p-methoxycinnamic acid or its derivative. Preferred derivatives are selected from 2-ethylhexyl-p-methoxycinnamate, ammonium-p-methoxycinnamate, sodium-p-methoxycinnamate, potassium-p-methoxycinnamate, or salts of primary, secondary or tertiary amines of p-methoxycinnamic acid and more preferably it is 2-ethylhexyl-p-methoxy cinnamate. A highly preferred aspect of the invention provides for incorporation of 0.1 % to 10 %, more preferably 0.1 to 5% by weight p-methoxycinnamic acid or its derivative. Most preferred p-methoxycinnamic acid derivative is 2-ethyl-hexyl-4-methoxycinnamate. Inclusion of p-methoxycinnamic acid derivative is especially useful since in addition to providing the known UV-B protection, the liquid emulsion or microemulsion composition ensures better stability of dibenzoylmethane derivative in the presence of p-methoxycinnamic acid derivative.

Useful inorganic sun-blocks may be preferably used in the present invention. These include, for example, zinc oxide iron oxide, silica such as fumed silica, and titanium dioxide.

Ultrafine titanium dioxide in either of its two forms, namely water-dispersible titanium dioxide and oil-dispersible titanium dioxide is especially suitable for the invention. Water-dispersible titanium dioxide is ultra-fine titanium dioxide, the particles of which are non-coated or which are coated with a material to impart a hydrophilic surface property to the particles. Examples of such materials include aluminium oxide and aluminium silicate.

Oil-dispersible titanium dioxide is ultrafine titanium dioxide, the particles of which exhibit a hydrophobic surface property, and which, for this purpose, can be coated with metal soaps such as aluminium stearate, aluminium laurate or zinc stearate, or with organosilicone compounds.

By "ultrafine titanium dioxide" is meant particles of titanium dioxide having a number average primary particle size of less than 100 nm, preferably 70 nm or less, more preferably from 10 to 40 nm and most preferably from 15 to 25 nm. By topical application to the skin of a mixture of both water-dispersible ultrafine titanium dioxide and oil-dispersible ultrafine titanium dioxide, synergistically enhanced protection of the skin against the harmful effects of both UV-A and UV-B rays is achievable.

Ultrafine titanium dioxide is the preferred inorganic sun-block agent as per this invention. The total amount of sun block that is preferably incorporated in the composition according to the invention is from 0.1 to 5% by weight of the composition.

The composition according to the invention may also comprise other diluents. The diluents act as a dispersant or carrier for other materials present in the composition, so as to facilitate their distribution when the composition is applied to the skin.

Diluents other than water can include liquid or solid emollients, solvents, humectants, thickeners and powders. Examples of each of these types of vehicle, which can be used singly or as mixtures of one or more vehicles, are as follows:

Emollients, such as stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate;

Solvents, such as ethyl alcohol, isopropanol, acetone, ethylene glycol monoethyl ether, diethylene glycol monobutyl ether, diethylene glycol monoethyl ether;

Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The cosmetically acceptable base is usually from 10 to 99.9%, preferably from 50 to 99% by weight of the composition, and can, in the absence of other cosmetic adjuncts, form the balance of the composition.

The composition of the invention may comprise a conventional deodourant base as the cosmetically acceptable carrier. By a deodorant is meant a product in roll-on or propellant medium which is used for personal deodorant benefit e.g. application in the under-arm or any other area which may or may not contain anti-perspirant actives.

Deodorant compositions can generally be in the form of gels, creams, and liquids and are dispensed using applicators appropriate to the physical characteristics of the composition.

Deodorant compositions which are delivered through roll-ons generally comprise a liquid carrier. Such liquid carrier can be hydrophobic or comprise a mixture of both hydrophilic and hydrophobic liquids. They may be in the form of an emulsion or a microemulsion. The liquid carrier or mixture of carriers often constitutes from 30 to 95% by weight of the composition and in many instances from 40 to 80%.

Anti-perspirants/deodorants skin care compositions of the invention may further include well known antiperspirant metal salts of aluminum, zinc, zirconium and zirconium aluminum mixtures of sulfates, chlorides, chlorohydroxides, tetrachlorohydrex glycinates, alums, formates, lactates, benzyl sulfonates, succinates, phenol sulfonates and the like. Typical levels of antiperspirant/deodorant agent are from about 0% to about 35%, preferably from about 0% to about 25% by weight of the composition. The composition may further include a complexing agent such as an organic acid or derivative thereof that are capable of forming complexes with the antiperspirant metallic salt. Examples of such complexing agents include, but are not limited to acetic acid, propionic acid, oxalic acid, malonic acid, succinic acid, tartaric acid, glycine and the like together with their cosmetically acceptable salts. Typical levels of complexing agent are from about 0% to about 15%, preferably from about 0% to about 10%, by weight of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

The invention is now further described by way of the following non-limiting examples.

**Examples**

Examples 1 and 2: Stability of sunscreen compositions of the invention in

comparison to conventional composition

A conventional sunscreen composition (example 1) and a composition as per the invention (example 2) were prepared.

The composition of example 1, in the form of a cream, is given in table 1. The cream composition was prepared as per the following process

An aqueous phase which comprises water, glycerine, potassium hydroxide and titanium dioxide were weighed and heated to a temperature in the range of 72 to 75 °C under constant stirring using a water bath. In a side pot-1, hystearic acid was charged and heated to 75 °C for melting. In a side pot-2, the oil phase comprising of cetyl alcohol, phenoxyethanol, isopropyl myristate, silicone oil and Parsol 1789 were weighed and heated to 75 °C. The molten hystearic acid was added to water at a temperature of 75 °C and the system was homogenized for 3 to 5 minutes using a Silverson mixer. The oil phase from side pot-2 was then added and again homogenized for 2 to 4 minutes. The emulsion was then cooled to the room temperature (about 25 °C) with slow mixing until the required viscosity was obtained.

| Ingredient | Wt% |
|---|---|
| Glycerine | 1.00 |
| Hysteric acid | 18.00 |
| Potassium hydroxide | 0.67 |
| Cetyl alcohol | 0.53 |
| Parsol 1789 | 0.40 |
| Phenoxy ethanol | 0.20 |
| Isopropyl myristate | 0.75 |
| Silicone oil | 0.50 |
| Titanium dioxide | 0.90 |
| Water | To 100 |

The composition of example 2 is given in table 2. The composition is in the form of a liquid transparent microemulsion. The microemulsion composition was prepared as per the following process.

To prepare the microemulsion, the sunscreen was first dissolved in the hydrophobic liquid in a beaker using a magnetic stirrer. Next weighed amounts of the emulsifier and co-emulsifier, were added to the beaker. Finally the required amount of water was added to the system. The components were blended either by gentle stirring (manually or by using a magnetic stirrer) to a get transparent microemulsion.

| Ingredient | Wt% |
|---|---|
| Isopropyl myristate (hydrophobic liquid) | 14.00 |
| Brij 35 (Emulsifier) | 28.00 |
| 1-butanol (co-emulsifier) | 28.00 |
| Parsol 1789 | 0.40 |
| Water | To 100 |

The stability of the formulation was measured using the following method.

The stability of the formulations was measured using SPF-290S (Optometrics Corporation).

A Transpore tape (3M) was used as the substrate to assess the stability of all the formulations. The transpore tape was stretched on a sample holder and 2mg/cm$^2$ of sample was applied on it, distributed uniformly as small dots using a syringe. Using parafilm as a finger cot, the sample was spread on the transpore uniformly swiping alternately, vertically and horizontally as per the protocol provided by the instrument manufacturers. The film was allowed to dry for fifteen minutes. After fifteen minutes the sample plate was exposed to UV lamp and a time based transmittance scan was done. This scan gives the transmittance across the wavelength range for a given sample at a single spot. The spot was continuously exposed to UV lamp and the transmittance scans were taken at interval of five minutes. The transmittance at 360 nm corresponds to the specific wavelength of Parsol 1789. The transmittance values at 360 nm were recorded and were used to obtain the percentage of Parsol 1789 remaining in the system, using the formula.

$$\% remaining = \frac{100 - T360_t}{100 - T360_{initial}} \times 100$$

The reference transmittance scan was obtained using a blank plate, with no sample on transpore tape.

The data on percent of Parsol 1789 remaining after exposure to UV radiation for examples 1 and 2 is shown in table 3.

| Time, minutes | % Parsol 1789 remaining | |
|---|---|---|
| | Example 1 | Example 2 |
| 0 | 100 | 100 |
| 10 | 74.4 | 90.0 |
| 20 | 67.0 | 78.4 |
| 25 | 64.8 | 72.7 |

The data in table 3 indicates that the UV- A sunscreen in a composition as per the invention (example 2) is more stable over a course of time as compared to a conventional composition (example 1).

Examples 3 to 7 Stability of sunscreen compositions (also containing UVB sunscreens) of the invention in comparison to conventional composition

Various other sunscreen microemulsion compositions as per the invention examples (4 to 7) were prepared and compared to a conventional cream composition (example 3). In all the examples 3 to 7, UV B sunscreen (Parsol MCX) was included in addition to a UV A sunscreen (Parsol 1789).

The composition of example 3 is as shown in table 4. This composition was prepared using a process similar to example 1.

| Ingredient | Wt% |
|---|---|
| Glycerine | 1.00 |
| Hysteric acid | 18.00 |
| Potassium hydroxide | 0.67 |
| Cetyl alcohol | 0.53 |
| Parsol 1789 | 0.40 |
| Parsol MCX | 0.75 |
| Phenoxy ethanol | 0.20 |
| Isopropyl myristate | 0.75 |
| Silicone oil | 0.50 |
| Titanium dioxide | 0.90 |
| Water | To 100 |

The compositions of examples 4 to 7 are shown in table 5

| | Example 4 | Example 5 | Example 6 | Example 7 |
|---|---|---|---|---|
| Ingredient | Ingredient, wt% | Ingredient, wt% | Ingredient, wt% | Ingredient, wt% |
| Emulsifier | Brij 97, 18.00 | AOT, 14.00 | C12EO3, 34.50 | Brij 35, 28.00 |
| Hydrophobic liquid | IPP, 31.30 | IPP, 56.52 | LLPO, 34.50 | IPM, 14.00 |
| Co-emulsifier | 1-butanol 18.00 | 1-butanol 7.73 | IPA 15.00 | 1-butanol 28.00 |
| Parsol 1789 | 0.40 | 0.40 | 0.40 | 0.40 |
| Parsol MCX | 0.75 | 0.75 | 0.75 | 0.75 |
| Water | To 100 | To 100 | To 100 | To 100 |

In the above table the following notations are used

IPP - Iso propyl palmitate
Brij 97 is a surfactant of the non-ionic polyethylene glycol monoethyl ether class
AOT is a surfactant of the anionic class and stands for aerosol OT which is sodium di-2-ethylhexyl sulfosuccinate.
C12EO3 (trioxyethylene dodecyl ether) is a surfactant of the non-ionic alkyl ethoxylates class
LLPO is light liquid paraffin oil.
IPA is iso propyl alcohol
IPM is iso propyl myristate

The liquid transparent microemulsion compositions of examples 4 to 7 were prepared using a process similar to that used for preparing example 2.
The stability of the compositions for examples 3 to 7 was measured using the method described for examples 1 and 2.
The data on percent of Parsol 1789 remaining after exposure to UV radiation for examples 3 to 7 is shown in table 6.

| Time, minutes | % Parsol 1789 remaining | | | | |
|---|---|---|---|---|---|
| | Example 3 | Example 4 | Example 5 | Example 6 | Example 7 |
| 0 | 100 | 100 | 100 | 100 | 100 |

(continued)

| Time, minutes | % Parsol 1789 remaining | | | | |
|---|---|---|---|---|---|
| | Example 3 | Example 4 | Example 5 | Example 6 | Example 7 |
| 10 | 64.9 | 88.1 | 90.1 | 96.0 | 96.9 |
| 20 | 55.1 | 75.3 | 81.3 | 86.1 | 89.8 |
| 30 | 51.1 | 66.5 | 74.4 | 74.7 | 81.8 |

The data in table 6 indicates that the UV- A sunscreen in compositions as per the invention (example 4 to 7) are more stable over time as compared to a conventional composition (example 3). Further, the microemulsion compositions of examples 4 to 7 were examined and found to be clear and transparent to the visible eye, thus confirming that the droplet sizes therein are smaller than 100 nanometers.

Examples 8 and 9: Comparison of microemulsion compositions as compared to emulsion compositions

Emulsion compositions (examples 8 and 9) were prepared which are shown in table 7 where the compositions of corresponding microemulsion compositions (examples 7 and 4) are also reiterated. By the corresponding microemulsions is meant that the compositions are close together on the phase diagram.

| | Example 7 | Example 8 | Example 4 | Example 9 |
|---|---|---|---|---|
| Ingredient | Ingredient, wt% | Ingredient, wt% | Ingredient, wt% | Ingredient, wt% |
| Emulsifier | Brij 35, 28.00 | Brij 35, 25.50 | Brij 97, 18.00 | Brij 97, 15.00 |
| Hydrophobic liquid | IPM, 14.00 | IPM, 18.00 | IPP, 31.30 | IPP, 32.00 |
| Co-emulsifier | 1-butanol 28.00 | 1-butanol 25.50 | 1-butanol 18.00 | 1-butanol 15.00 |
| Parsol 1789 | 0.40 | 0.40 | 0.40 | 0.40 |
| Parsol MCX | 0.75 | 0.75 | 0.75 | 0.75 |
| Water | To 100 | To 100 | To 100 | To 100 |

The % transmittance of the various compositions listed in table 7 were measured using a procedure as described below.

The transmittance measurements were done using SPF-290S (Optometrics Corporation). A Transpore tape (3M) was used as the substrate to assess the stability of all the formulations. The transpore tape was stretched on a sample holder and $2mg/cm^2$ of sample was applied on it, distributed uniformly as small dots using a syringe. Using parafilm as a finger cot, the sample was spread on the transpore uniformly swiping alternately, vertically and horizontally as per the protocol provided by the instrument manufacturers. The film was allowed to dry for fifteen minutes. After fifteen minutes the sample plate was exposed to UV lamp and a transmittance scan was done. This scan gives the transmittance as a function of wavelength for a given sample. For a single plate the instrument scans six different spots. The same was reported for 2 more plates. The data reported is thus an average over 18 reading.

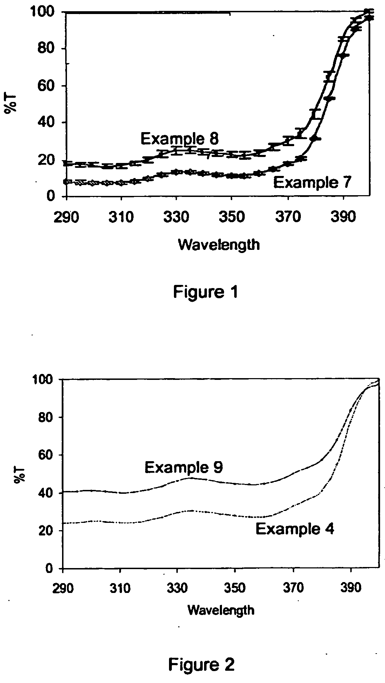

The reference transmittance scan was obtained using a blank plate, with no sample on transpore tape. The data on % transmittance over a wide wavelength range (290 to 400 nm) is shown in figure 1 and 2. The data indicates that the % transmittance of microemulsion compositions of the invention (examples 7 and 4) are lower as compared to emulsion compositions of the invention (examples 8 and 9) thereby affording better photoprotection.

Examples 10 to 12: Stability of sunscreen compositions within the invention as compared to those outside the invention

Compositions as shown in table 8 were prepared and the transmittance of the various compositions were measured similar to those of compositions 7 to 9. The data was measured at zero time and after 30 minutes of exposure to UV radiation. The data on % transmittance is shown in table 8.

|  | Example 10 | Example 11 | Example 12 |
|---|---|---|---|
| Ingredient | wt% | wt% | wt% |
| Emulsifier, Brij-35 | 28.50 | 15.00 | 5.00 |
| Hydrophobic liquid, IPM | 14.00 | 18.00 | 18.00 |
| Co-emulsifier, 1-butanol | 28.00 | 25.50 | 25.50 |
| Parsol 1789 | 1.20 | 1.20 | 1.20 |
| Parsol MCX | 2.25 | 2.25 | 2.25 |
| Water | To 100 | To 100 | To 100 |
| %Transmittance, zero time | 13.4 | 35.1 | 71.3 |
| % Transmittance, 30 minutes | 29.2 | 43.4 | 78.4 |

The data in table 8 indicates that compositions within the invention (examples 10 and 11) which have emulsifier concentration within the claimed range provide good photoprotection (low values of % transmission) as compared to a composition outside the invention (example 12 which has emulsifier concentration lower than the claimed range).

**Claims**

1. A photostable sunscreen composition in microemuision format with dispersed phase droplets in the size range of 4 to 100 nm, the photostable sunscreen composition comprising :

   a. 0.1 % to 10 % by weight dibenzoylmethane or its derivative;
   b. 10 to 40% by weight an emulsifier;
   c. 5 to 40% by weight a co-emulsifier selected from the group consisting of C2 to C8 straight or branched chain alcohol, polyhydric alcohol and C5 to C8 low molecular weight fatty acid;
   d. 10 to 50% by weight of a hydrophobic liquid having a log P value at 25 degrees centigrade greater than 3 and a water solubility at 25 degrees centigrade less than 0.1 % by weight; and
   e. 10 to 50% by weight water,

   wherein said emulsifier is a nonionic surfactant;
   wherein the dibenzoylmethane derivative is selected from 4-tert-butyl-4'-methoxydibenzoylmethane, 2-methyldibenzoylmethane, 4-methyl-dibenzoylmethane, 4-isopropyldibenzoylmethane, 4-tert-butyldibenzoylmethane, 2, 4-dimethyldibenzoylmethane, 2, 5-dimethyldibenzoylmethane, 4, 4'-diisopropyl-dibenzoylmethane, 2-methyl-5-isopropyl-4'-methoxydibenzoylmethane, 2-methyl-5-tert-butyl-4-methoxydibenzoylmethane,2, 4-dimethyl-4'-methoxy-dibenzoylmethane or 2, 6-dimethyl-4-tert-butyl-4'-methoxy-dibenzoylmethane.

2. A composition as claimed in claim 1 wherein said non-ionic surfactant is selected from the group consisting of polyethylene glycol monoethyl ether, polysorbate, alcohol ethoxylates, and polyoxyethylene esters of fatty acid.

3. A composition as claimed in claim 1 or claim 2 comprising 10 to 30% by weight emulsifier.

4. A composition as claimed in any one of the preceding claims wherein said co-emulsifier is selected from the group consisting of ethanol, propanol, butanol, polyethylene glycol, propylene glycol and caprylic acid.

5. A composition as claimed in any one of the preceding claims comprising 5 to 30% by weight co-emulsifier.

6. A composition as claimed in any one of the preceding claims wherein said hydrophobic liquid is selected from the group consisting of hydrocarbons, fatty alcohols, fatty alcohol esters, triglycerides or fatty acids and silicone oils.

7. A composition as claimed in claim 6 wherein said hydrophobic liquid is selected from the group consisting of light liquid paraffin oil, iso propyl myristate, iso propyl palmitate and silicone oil.

8. A composition as claimed in claim 1 wherein the microemulsion is in a transparent liquid form.

9. A composition as claimed in any one of the preceding claims wherein the dibenzoyl methane derivative is 4-tert.-butyl-4'-methoxydibenzoylmethane.

10. A composition as claimed in any one of the preceding claims comprising 0.1 % to 10 % by weight p-methoxycinnamic acid or its derivative;

11. A composition as claimed in claim 10 wherein said p-methoxycinnamic acid derivative is 2-ethyl-hexyl-4-methoxycinnamate.

**Patentansprüche**

1. Lichtstabile Sonnenschutzzusammensetzung im Mikroemulsionsformat mit dispergierten Phasentröpfchen in dem Größenbereich von 4 bis 100 nm, wobei die lichtstabile Sonnenschutzzusammensetzung umfasst:

   a. 0,1 bis 10 Gewichts-% Dibenzoylmethan oder sein Derivat,
   b. 10 bis 40 Gewichts-% eines Emulgators,
   c. 5 bis 40 Gewichts-% eines Co-Emulgators, ausgewählt aus der aus geradkettigem oder verzweigtkettigem C2- bis C8-Alkohol, mehrwertigem Alkohol und einer C5- bis C8-Fettsäure niedrigen Molekulargewichts bestehenden Gruppe,
   d. 10 bis 50 Gewichts-% einer hydrophoben Flüssigkeit mit einem log P-Wert bei 25 Grad Celsius von größer als 3 und einer Wasserlöslichkeit bei 25 Grad Celsius von weniger als 0,1 Gewichts-% und
   e. 10 bis 50 Gewichts-% Wasser,

   wobei der Emulgator ein nichtionisches Tensid ist,
   wobei das Dibenzoylmethanderivat unter 4-tert-Butyl-4'-methoxydibenzoylmethan, 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan oder 2,6-Dimethyl-4-tert-butyl-4'-methoxydibenzoylmethan ausgewählt ist.

2. Zusammensetzung nach Anspruch 1, wobei das nicht-ionische Tensid aus der aus Polyethylenglycolmonoethylether, Polysorbat, Alkoholethoxylat und Polyoxyethylenester von Fettsäure bestehenden Gruppe ausgewählt ist.

3. Zusammensetzung nach Anspruch 1 oder 2, umfassend 10 bis 30 Gewichts-% Emulgator.

4. Zusammensetzung nach irgendeinem der vorhergehenden Ansprüche, wobei der Coemulgator aus der aus Ethanol, Propanol, Butanol, Polyethylenglycol, Propylenglycol und Caprylsäure bestehenden Gruppe ausgewählt ist.

5. Zusammensetzung nach irgendeinem der vorhergehenden Ansprüche, umfassend 5 bis 30 Gewichts-% Coemulgator.

6. Zusammensetzung nach irgendeinem der vorhergehenden Ansprüche, wobei die hydrophobe Flüssigkeit aus der aus Kohlenwasserstoffen, Fettalkoholen, Fettalkoholestern, Triglyceriden oder Fettsäuren und Siliconölen bestehenden Gruppe ausgewählt ist.

7. Zusammensetzung nach Anspruch 6, wobei die hydrophobe Flüssigkeit aus der aus hellem flüssigem Paraffinöl, Isopropylmyristat, Isopropylpalmitat und Siliconöl bestehenden Gruppe ausgewählt ist.

8. Zusammensetzung nach Anspruch 1, wobei die Mikroemulsion in transparenter flüssiger Form vorliegt.

9. Zusammensetzung nach irgendeinem der vorhergehenden Ansprüche, wobei das Dibenzoylmethanderivat 4-tert-Butyl-4'-methoxydibenzoylmethan ist.

10. Zusammensetzung nach irgendeinem der vorhergehenden Ansprüche, umfassend 0,1 bis 10 Gewichts-% p-Methoxyzimtsäure oder ihr Derivat.

11. Zusammensetzung nach Anspruch 10, wobei das p-Methoxyzimtsäurederivat 2-Ethyl-hexyl-4-methoxyzinnamat ist.

**Revendications**

1.  Composition d'écran solaire photostable en format de microémulsion avec des gouttelettes de phase dispersée ayant une taille située dans la plage allant de 4 à 100 nm, la composition d'écran solaire photostable comprenant :

    a. 0,1 % à 10 % en poids de dibenzoylméthane ou de son dérivé ;
    b. 10 à 40 % en poids d'un émulsionnant ;
    c. 5 à 40 % en poids d'un co-émulsionnant choisi dans le groupe constitué par les alcools à chaîne droite ou ramifiée en $C_2$ à $C_8$, les alcools polyvalents et les acides gras de faible masse moléculaire en $C_5$ à $C_8$ ;
    d. 10 à 50 % en poids d'un liquide hydrophobe ayant une valeur de log P à 25°C supérieure à 3 et une solubilité dans l'eau à 25°C inférieure à 0,1 % en poids ; et
    e. 10 à 50 % en poids d'eau,

    dans laquelle ledit émulsionnant est un tensioactif non-ionique ;
    dans laquelle le dérivé de dibenzoylméthane est choisi parmi le 4-tert-butyl-4'-méthoxydibenzoylméthane, le 2-méthyldibenzoylméthane, le 4-méthyldibenzoylméthane, le 4-isopropyldibenzoylméthane, le 4-tert-butyldibenzoylméthane, le 2,4-diméthyldibenzoylméthane, le 2,5-diméthyldibenzoylméthane, le 4,4'-düsopropyldibenzoylméthane, le 2-méthyl-5-isopropyl-4'-méthoxydibenzoylméthane, le 2-méthyl-5-tert-butyl-4'-méthoxydibenzoylméthane, le 2,4-diméthyl-4'-méthoxydibenzoylméthane et le 2,6-diméthyl-4-tert-butyl-4'-méthoxydibenzoylméthane.

2.  Composition selon la revendication 1, dans laquelle ledit tensioactif non-ionique est choisi dans le groupe constitué par l'éther monoéthylique de polyéthylèneglycol, le polysorbate, l'alcool éthoxylé, et les esters d'acides gras polyoxyéthylénés.

3.  Composition selon la revendication 1 ou la revendication 2, comprenant 10 à 30 % en poids d'émulsionnant.

4.  Composition selon l'une quelconque des revendications précédentes, dans laquelle ledit co-émulsionnant est choisi dans le groupe constitué par l'éthanol, le propanol, le butanol, le polyéthylèneglycol, le propylèneglycol et l'acide caprylique.

5.  Composition selon l'une quelconque des revendications précédentes, comprenant 5 à 30 % en poids de co-émulsionnant.

6.  Composition selon l'une quelconque des revendications précédentes, dans laquelle ledit liquide hydrophobe est choisi dans le groupe constitué par les hydrocarbures, les alcools gras, les esters d'alcools gras, les triglycérides, les acides gras et les huiles siliconées.

7.  Composition selon la revendication 6, dans laquelle ledit liquide hydrophobe est choisi dans le groupe constitué par l'huile de paraffine liquide légère, le myristate d'isopropyle, le palmitate d'isopropyle et l'huile de silicone.

8.  Composition selon la revendication 1, dans laquelle la microémulsion est sous une forme liquide transparente.

9.  Composition selon l'une quelconque des revendications précédentes, dans laquelle le dérivé de dibenzoylméthane est le 4-tert-butyl-4'-méthoxydibenzoylméthane.

10. Composition selon l'une quelconque des revendications précédentes, comprenant 0,1 % à 10 % en poids d'acide p-méthoxycinnamique ou de son dérivé.

11. Composition selon la revendication 10, dans laquelle ledit dérivé d'acide p-méthoxycinnamique est le 4-méthoxy-cinnamate de 2-éthylhexyle.

Figure 1

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5985251 A
- US 5576354 A, L'Oreal
- WO 0137798 A
- WO 2008022946 A

**Non-patent literature cited in the description**

- **HIROSHI CHUMAN ; ATSUSHI MORI ; HIDEJI TANAKA.** Prediction of the 1-Octanol/H2O Partition Coefficient, Log P, by Ab Initio MO Calculations: Hydrogen-Bonding Effect of Organic Solutes on Log P. *Analytical Sciences,* September 2002, vol. 18, 1015-1020